# EUROPEAN PATENT APPLICATION

(11) **EP 2 946 728 A1**
(43) Date of publication of application: **25.11.2015**
(21) Application number: 15168081.6
(22) Date of filing: 19.05.2015
(51) Int. Cl.: A61B 5/00, A61B 5/053, A61B 5/08

(54) **ELECTRODE ARRAY WITH PERFORATED BACKING**

(30) Priority: 20.05.2014 GB 201408962; 20.05.2014 GB 201408964
(71) Applicant: SLE Limited, South Croydon, Surrey CR2 6PL (GB)
(72) Inventor: Boydell, Juliet Marie, South Croydon, Surrey CR2 6PL (GB); Mazela, Jan Lukasz, 61028 Poznan (PL)
(74) Representative: Andrews, Paul David

(57) **Abstract**

This invention relates to an electrode array suitable for measuring impedance across a patient's lung, the array comprising: (i) two or more electrodes which are mounted on a substrate and are connectable to an electrical impedance segmentography monitor, and (ii) an adhesive surface for attaching the electrodes to the patient's skin. This invention also relates to an electrical impedance segmentography monitoring system comprising: (i) an electrical impedance segmentography monitor, and (ii) two electrode arrays as described above which are connectable to the monitor.

## Description

This invention relates to an electrode array for use in electrical impedance segmentography (EIS). In particular, the electrode array is for neonatal use, ie for use on newborn babies (normally, although not exclusively, up to 28 days after birth).

### Background

Electrical Impedance Segmentography (EIS) is a painless, low cost, noninvasive and radiation-free method which allows the user to continuously record the distribution of air and fluids in parts of the human body.

EIS is used (particularly in neonates) to monitor the impedance across the four quadrants of the lung. This is considered to be representative of the equivalent ventilation and shows changes in volume for each of these quadrants. Regional changes of lung ventilation, such as alveolar collapse and atelectasis, pneumothorax, thoracic effusions, misplacement of tracheal tubes or surfactant can be diagnosed by x-ray, but cannot be monitored continuously in clinical routine at bedside in NICUs at present. In relation to this invention, the term "lung quadrant" is used to mean a portion of each lung defined by dividing each lung approximately into upper and lower halves by volume (ie resulting in four "quadrants" when considering both left and right lungs together). The term "upper" is used to mean the quadrant closer to the patient's head, and the term "lower" are used to mean the quadrant closer to the patient's feet.

Therefore methods to monitor regional ventilation of spontaneously breathing infants and especially of mechanically ventilated infants at bedside are required.

Inhomogeneous distribution of air and ventilation between the right and left lungs, as well as within each lung, remains a major problem in neonatal intensive care. It constitutes a therapeutic dilemma, since increasing ventilatory support also increases the inhomogeneity and gas exchange disturbances. Alveolar collapse or overdistension of the lungs is associated with ventilation/perfusion mismatch.

It is known to use x-rays on a regular basis to assess the regional distribution of ventilation, which largely affects the capability of the lung to exchange gases. For many therapeutic measures, such as adjustments of ventilator settings, recruitment manoeuvres, patient positioning, lung suction, and pneumothoraces, it is highly beneficial to get immediate feedback on a breath-by-breath basis to assess the efficiency of the measure. EIS, particularly the Angelie EIS System produced by SLE Limited, allows the production of images, real-time impedance curves and derived parameters which can provide this feedback immediately and in real-time.

Newborn babies are small and have delicate anatomies, and so insertion of a tube into the airway tends to be a more complicated process than inserting a tube into an adult. Possible risks of a neonatal intubation include damage to areas of the body from the tube, or insertion of the tube into the wrong area, therefore affecting the oxygen getting into the body. Tubes may also become blocked, with blood or mucus, or may fall out of place. Once intubated, the carer has to perform safety checks to confirm that sufficient oxygen is getting to the baby. EIS allows the user to see where the air is going in the lungs and to monitor any changes in its distribution.

Babies born prematurely often have respiratory systems that are highly fragile and still developing or in arrested development. EIS can help monitor the normal distribution of gas within a baby's lungs and give advanced warning of potential problems. Forced respiration combined with the newborn's fragile breathing organs can cause ruptures in the lungs, alveoli or both. Although surfactant may keep alveoli supple during flexing, constant mechanical ventilation can overwork even well-coated air sacs, causing tears. This is the most common reason for pneumothorax in newborns. If the alveoli don't break, the lungs themselves might tear and develop holes if stressed by the machine ventilator.

Further description of EIS can be found in the following references: (i) Electrical Impedance Segmentography, Regional Lung Ventilation in Infants, Judith C Weinknecht, June 2009; (ii) Continuous Noninvasive Monitoring of Tidal Volumes by Measurement of Tidal Impedance in Neonatal Piglets, Kurth et al, PLoS ONE June 2011 v6.6; (iii) Continuous Noninvasive Monitoring of Lung Recruitment during High Frequency Oscillatory Ventilation by Electrical Impedance Measurement, An Animal Study, Burkhardt et al, Neonatology, May 2012.

A problem with current EIS systems relates to the attachment of the electrodes to the body of the newborn. In order to obtain correct monitoring of the newborn's lung function, ten electrodes are normally attached to its torso. Five electrodes are attached to the newborn's chest, and five to its back. Four of each of the two sets of five electrodes measure lung function whereas one of each of the two sets of five electrodes is a reference electrode. The electrodes are normally attached such that the electrodes on the newborn's back are a mirror image of those on the chest. In addition, the electrodes are usually attached in the form of a cross (X), with one electrode (for measuring lung function) at each of the four extremities of the cross, and one reference electrode at the centre of the cross.

It is time-consuming to apply ten individual electrodes to a newborn. In addition, it may be necessary to apply and remove the electrodes several times during a newborn's treatment. The application and removal of the electrodes can cause stress to the newborn, particularly if they have other health problems.

Any method of ameliorating these problems must also meet several other competing requirements. For example, the electrodes should not interfere with other monitoring equipment such as chest drains, belly button attachments, temperature sensor patches, transcutaneous O₂ and CO₂ probes, ECG, auscultation and heart ultrasound. Furthermore, since a newborn's skin is especially sensitive, the electrodes should minimise damage to the skin. In addition, the device needs to be comfortable for the newborn, which may be lying on its front, side or back. The electrodes should also minimise restriction to the movement or breathing of the newborn.

From a user's point of view, any method of ameliorating the problems should allow quick application of the electrodes to the patient. It should also be easy to use and easy to understand how to use. It is advantageous if the application process can be carried out by one person.

### Summary of the invention

This invention relates to an electrode array suitable for adhesion to a patient's skin, the array comprising:
(i) two or more electrodes which are mounted on a substrate and are connectable to an electrical impedance segmentography monitor,
(ii) an adhesive surface for attaching the electrodes to the patient's skin.

In this way, several electrodes can be applied to a patient's skin in a single step, thereby reducing any distress caused to a patient during the application process.

In relation to this invention, the word "proximal" is used to refer to the side of the electrode array that is to be applied to the patient's body. The word "distal" is used to the side of the electrode array that faces away from the patient's body.

The number of electrodes in the array can vary. Preferably, at least one of the electrodes is suitable for measuring the impedance across a patient's lung. Preferably, at least one of the electrodes (most preferably one) is suitable for use as a reference electrode, ie an electrode which does not measure the impedance across a patient's lung. In a preferred embodiment the electrode array comprises five electrodes. By providing five electrodes, four can be used to monitor the impedance across the four quadrants of the patient's lung. The fifth electrode can be used as the reference electrode.

It is preferred that the adhesive surface is provided on and/or around each of the electrodes. This is so that the electrodes can be attached to the patient's skin. Preferably, the adhesive surface comprises a hydrogel adhesive. Hydrogel adhesives can provide good adhesion, strength and flexibility whilst also being air and water permeable.

In some embodiments, the electrodes are connected to wires or printed tracks which are connectable to an electrical impedance segmentography monitor. In some embodiments, printed tracks are preferred in order to provide improved comfort for the patient. This is because printed tracks are flatter than wires and can therefore be more comfortable for a patient to lie on.

In some embodiments, the substrate is a flexible backing material. The electrodes are preferably mounted on the proximal side of the flexible backing material. This flexible backing material can provide strength to the electrode array. Preferably, the flexible backing material comprises a polymer foam, more preferably a polyurethane foam. It is preferred that the flexible backing material is perforated. The perforations can improve the flexibility of the backing material, increasing comfort for the patient. The perforations can be in the form of one or more apertures in the flexible backing material. The perforations can take a variety of shapes, including linear apertures and circular apertures. Preferably, the electrodes are arranged on the flexible backing material such that, in use, each of four of the electrodes has a position on the patient's skin proximal to each of the four quadrants of the patient's lung. Preferably, in use, a further electrode has a position on the patient's skin approximately equidistant from these four electrodes. Preferably the flexible backing material comprises an adhesive liner which is removably attached to its proximal surface.

In some embodiments, the flexible backing material is x-shaped, with an electrode mounted proximal to each of the four extremities of the x-shape, and one electrode mounted approximately at the centre of the cross.

This invention also relates an electrical impedance segmentography monitoring system comprising:
(i) an electrical impedance segmentography monitor, and
(ii) two electrode arrays as described above which are connectable to the monitor.

This invention will be further described by reference to the following Figures which are not intended to limit the scope of the invention claimed, in which:
**Figure 1** shows a proximal view of an electrode array according to a first embodiment of the invention with the connecting wires grouped as a single connection.
**Figure 2** shows a proximal view of a further version of the first embodiment of the invention with individual connecting wires.

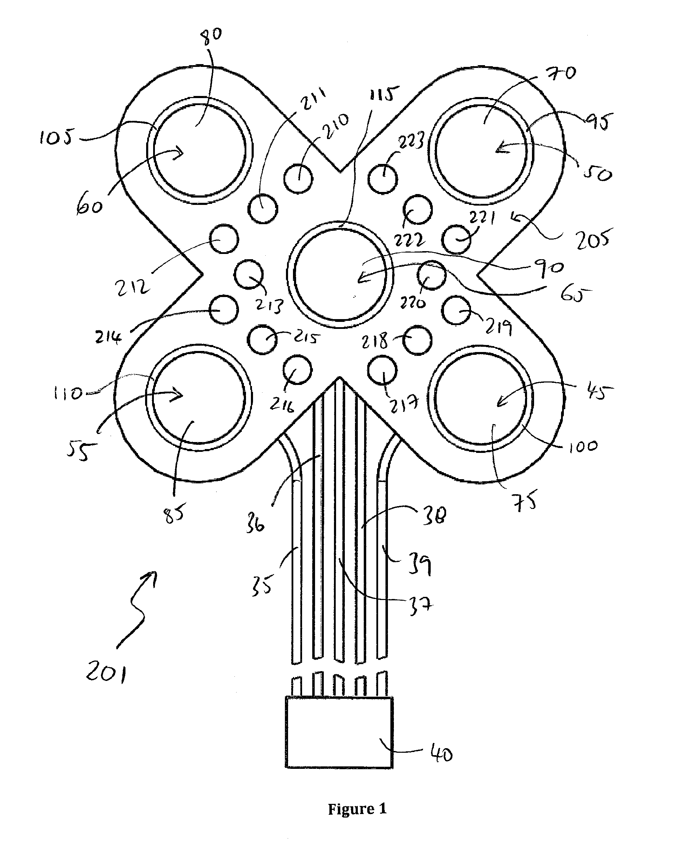

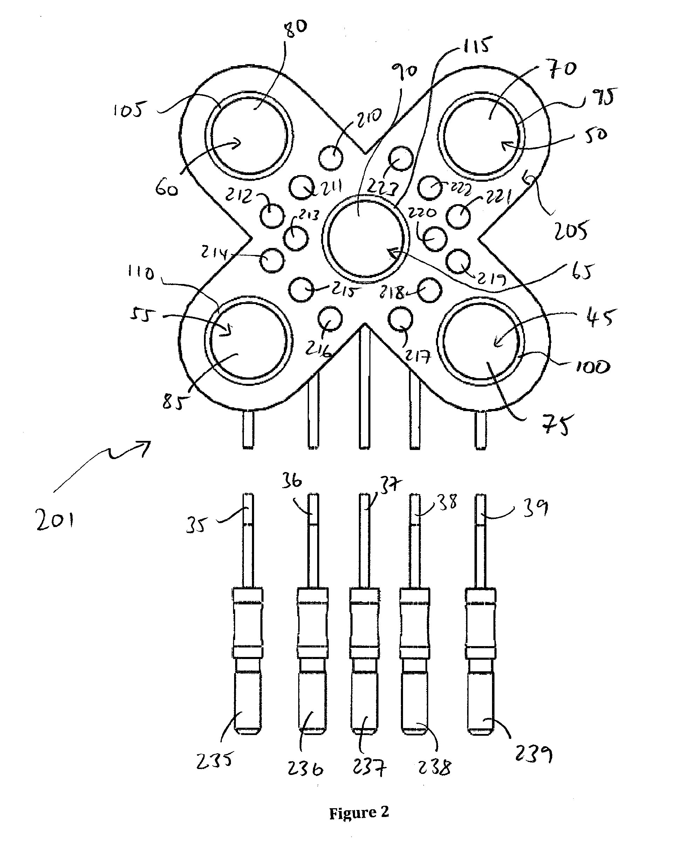

Figures 1 and 2 depict an electrode array 201 according to a first embodiment of the invention. Electrodes 45, 50, 55, 60, 65 are mounted on the proximal side of flexible backing material 205 which, prior to use, is covered in a removable one-piece adhesive liner (not shown). A two section adhesive liner could also be used. Flexible backing material 205 has a general x-shape. The electrodes 45, 50, 55, 60, 65 are arranged such that an electrode 45, 50, 55, 60 is provided proximal to each of four extremities of the x-shaped flexible backing material 205, and that one electrode 65 is provided approximately at the centre of the x-shaped flexible backing material 205.

As shown in Figures 1 and 2, five wires 35, 36, 37, 38, 39 are provided, each of which is at one end connected to an electrode 45, 50, 55, 60, 65 respectively (connections not shown) and at the other end connected to adapter 40. Adapter 40 is shaped so that it can be connected to an electrical impedance segmentography monitor (not shown).

As shown in Figures 1 and 2, each electrode 45, 50, 55, 60, 65 has a proximal circular face 70, 75, 80, 85, 90 which in use contacts the patient's skin. Surrounding each proximal circular face 70, 75, 80, 85, 90 is an adhesive surface 95, 100, 105, 110, 115 so that each electrode 45, 50, 55, 60, 65 can be attached to a patient's skin.

The flexible backing material 205 is additionally provided with perforations 210, 211, 212, 213, 214, 215, 216, 217, 218, 219, 220, 221, 222, 223. The perforations can take a variety of shapes and positions on the flexible backing material 205, and there can be various numbers of them. In the embodiment depicted in Figures 1 and 2, there are fourteen circular apertures which are provided between the extremity electrodes 45, 50, 55, 60 and the centre electrode 65.

The two versions of the first embodiment shown in Figures 1 and 2 differ in that in Figure 1 wires 35, 36, 37, 38, 39 are connected to adapter 40 in order to provide a single connection to an electrical impedance segmentography monitor (not shown). In contrast, in Figure 2 wires 35, 36, 37, 38, 39 end in plugs 235, 236, 237, 238, 239 respectively. Each plug 235, 236, 237, 238, 239 is then connectable individually to an electrical impedance segmentography monitor (not shown).

## Claims

1. An electrode array suitable for measuring impedance across a patient's lung, the array comprising:
(i) two or more electrodes which are mounted on a substrate and are connectable to an electrical impedance segmentography monitor,
(ii) an adhesive surface for attaching the electrodes to the patient's skin

2. An electrode array as claimed in claim 1 comprising five electrodes.

3. An electrode array as claimed in claim 2, wherein the electrodes are arranged on the substrate such that, in use, each of four of the electrodes has a position on the patient's skin proximal to each of the four quadrants of the patient's lung and a fifth electrode has a position on the patient's skin approximately equidistant from these four electrodes

4. An electrode array as claimed in any one of the preceding claims, wherein the adhesive surface is provided on and/or around each of the electrodes.

5. An electrode array as claimed in any one of the preceding claims, wherein the electrodes are connected to wires or printed tracks which are connectable to an electrical impedance segmentography monitor.

6. An electrode array as claimed in any one of the preceding claims, wherein the substrate is a flexible backing material.

7. An electrode array as claimed in claim 6, wherein the flexible backing material is a polyurethane foam.

8. An electrode array as claimed in either claim 6 or claim 7, wherein the flexible backing material is perforated.

9. An electrode array as claimed in any one of claims 6-8, wherein the flexible backing material is x-shaped, with an electrode mounted proximal to each of the four extremities of the x-shape, and one electrode mounted approximately at the centre of the cross.

10. An electrode array as claimed in claim 9, wherein the perforations are in the form of linear apertures positioned between the electrodes.

11. An electrical impedance segmentography monitoring system comprising:
(i) an electrical impedance segmentography monitor, and
(ii) two electrode arrays as claimed in any one of the preceding claims which are connectable to the monitor.
